# EUROPEAN PATENT SPECIFICATION

(11) **EP 1 545 795 B1**
(45) Date of publication and mention of the grant of the patent: **17.12.2008**
(21) Application number: 03797761.8
(22) Date of filing: 27.08.2003
(51) Int. Cl.: B05B 11/00, A61F 9/00

(54) **DISPENSING APPARATUS AND METHOD FOR LIQUID PRODUCTS, PARTICULARLY MEDICINAL PRODUCTS**
ABGABEVORRICHTUNG UND -VERFAHREN FÜR FLÜSSIGE PRODUKTE, INSBESONDERE MEDIZINISCHE PRODUKTE
APPAREIL ET PROCEDE DE DISTRIBUTION POUR PRODUITS LIQUIDES, NOTAMMENT POUR PRODUITS MEDICAUX

(30) Priority: 23.09.2002 SE 0202800
(43) Date of publication of application: 29.06.2005
(73) Proprietor: Pfizer Health AB, 112 87 Stockholm (SE)
(72) Inventor: PAVLU, Bohdan, S-131 41 Nacka (SE); HIMBERT, Hans, S-168 56 Bromma (SE); PECLAT, Christian, CH-2000 Neuchâtel (CH); GREMION, Emmanuel, CH-1646 Echarlens (CH); SIEGFRIED, Daniel, CH-3012 Bern (CH); SAURER, Alain, CH-2000 Neuchâtel (CH); FONTANNAZ, Joel, CH-3097 Liebefeld (CH)
(74) Representative: Bridle, Andrew Barry
(86) International application number: PCT/SE2003/001326
(87) International publication number: WO 2004/026489

(56) References cited:
- EP-A2- 0 823 246
- WO-A1-90/13497
- DE-A1- 19 948 462
- US-A- 4 623 337
- US-A- 5 190 191
- US-A- 5 944 702

## Description

The present invention concerns a dispensing apparatus for liquid products, particularly medicinal products, such as an ophthalmic solution.

Although the principles of the present invention may have utility in many areas, for convenience it will be described mainly in connection with liquid treatment of eyes. Typically the medical preparation has to be delivered in a fairly well defined volume to assure a specified dose to be delivered or absorbed. A large surplus cannot be allowed due to improper systemic physiological effects from absorbency in non-target tissues or drainage of excess amounts th rough the tear channel into the throat cavity or the inconveniences caused by overflow on face and clothes. Also price considerations apply for expensive medications. As an example, the treatment of glaucoma requires frequent daily administrations of e.g. prostaglandins, beta-blockers or other expensive active ingredients, all having other then the desired pressure relieving action when absorbed by other body tissues than the eye. Small volume dosing is negatively affected by even small uncontrolled or dead spaces in delivery equipments used. Moreover, medical preparation components may be sensitive to degradation or absorption at prolonged exposure to materials and extended surfaces present in delivery devices. Similar considerations apply for sterility preservation. With regard to stream quality, proper administration of small amounts is complicated by the fact that the active ingredients cannot enter the eye but through the limited area of the cornea. It is also necessary that the entire dose can be delivered before the triggered blink reflex closes the eyelid.

A large number of devices are already known for applying a determined quantity of a liquid medicinal product onto a part of the body, such as an ophthalmic solution on the surface of the eye. These devices generally rely on the principle of a syringe which can be either pre-filled with a determined quantity of liquid, or graduated to suck up said quantity of liquid contained in a separate bottle, or connected to a fixed receptacle in permanent communication with the dosing chamber of the syringe, as is described for example in one of the embodiments of US Patent No. 4,623,337. It will be observed that permanently feeding the dosing chamber from the receptacle via gravity means that neither the precision of the quantity of liquid to be ejected, nor the sterility thereof can be guaranteed. In these devices, the pressure exerted on the plunger, manually or automatically, is generally exerted in the same direction as that of the liquid jet, as is described for example in International Patent Application No. WO 92/20455, which discloses the features of the preamble of claim 1.

The direction of the jet can sometimes be deviated by bent conduits, but it is then difficult to control the force with which the jet reaches its target. A device of this kind is like, for example, that disclosed in French Patent No. FR 2 647 757 for food products or cosmetics in liquid or paste-like form, for which respecting a given ejection pressure is of no importance.

In the case of a an ophthalmic solution, it is, however, very important not only to control very precisely the dose to be ejected for obvious reasons of safety and efficacy of the treatment, but also in order to be able to control the impact pressure of the liquid jet on the eye, which certain devices attempt to achieve by using an eyepiece or a spacing member applied to the periphery of the target to impose a fixed distance with respect to the liquid ejection orifice, as is disclosed for example in US Patent Nos. 4,623,337 and 5,836,911. It will be observed however that these devices do not always allow the impact force of the liquid jet to be reproduced when the pressure is exerted directly on the plunger manually.

Thus, the dispensing apparatuses of the prior art provide individual solutions to particular problems, but none of them allows all of the aforementioned problems to be simultaneously resolved.

The object of the present invention is thus to provide a dispensing method and device capable of avoiding the problems discussed above. More particularly an object is to provide a method and device system capable of ejecting, e.g. with a new design of the plunger head leaving practically no ullage, a precise dose of liquid, such as an ophthalmic solution, with an adjustable impact pressure on the target and the dose and impact pressure being independent of the way in which the pressure is exerted on the actuator. The apparatus according to the invention includes a mechanism allowing sterility conditions to be improved, given that the receptacle is only in communication with the dosing chamber except for a brief moment during ejection when it is placed in communication with the external environment for a few tenths of a second, during which time the pressure equilibrium is achieved by replacing the sucked up liquid with air. In addition the system allows uncontrolled and adead spaces to be kept to a minimum. The apparatus is further very easy to use in particular for an ophthalmic solution.

These and other objects are reached by the characteristics set forth in the appended patent claims.

The movement of the actuator is preferably substantially perpendicular to the direction of ejection of the liquid, such that the pressure exerted on the actuator cannot modify the distance with respect to the target, for example the eye in the case of an dispensing ophthalmic solution.

According to a first embodiment, the mobile element is formed by a drum provided, on its flanks, with studs rotatably mounted in the two shells of the housing, and housing in its diametral part an assembly formed by the dosing chamber, the plunger and the return spring.

At the start of pressure on the actuator, the drum occupies a first filling position in which the orifice of the dosing chamber is opposite the receptacle feed nozzle. By continuing to press on the actuator, the drum rotates through an angle α to occupy a second ejection position in which the orifice of the dosing chamber is opposite the through passage of the housing.

In a second embodiment, the dosing chamber is formed in a unit secured to the frame, and the mobile element is formed by a mobile valve, held in the rest position by a return spring. At the start of pressure on the actuator, the valve occupies a first position for filling the dosing chamber through a channel formed in the thickness of said valve placing the orifice of the dosing chamber in communication with the receptacle nozzle. By continuing to press on the actuator the valve is brought into a second ejection position in which the orifice of the dosing chamber is placed in communication with the exterior through a hole of the valve located opposite the through passage of the frame.

In both embodiments, the actuator is returned to the rest position by resilient return means, wound by the travel of the plunger during the filling and ejection phases. In these two embodiments, in order to further increase the conditions of sterility, the actuator can include a panel blocking the through passage of the housing or frame from the exterior in the rest position, said panel including an orifice brought to face said through passage in the ejection position.

Other features and advantages of the present invention will appear more clearly upon reading embodiment examples, given purely by way of non-limiting illustration, with reference to the annexed drawings, in which:
- Figure 1 shows a perspective view of a dispensing apparatus according to the invention without the external cover;
- Figure 2 shows a cross-section of the apparatus of Figure 1, along the arrows II-II parallel to the base of the apparatus;
- Figure 3 shows an exploded perspective view of the apparatus of Figure 1;
- Figure 4 shows a side view of the apparatus of Figure 1 in which one shell of the housing and the drum have been removed;
- Figure 5 shows a cross-section along the line V-V of Figure 2, of the mechanism assembly in the rest position;
- Figure 6 corresponds to the suction phase of a determined quantity of liquid to be ejected;
- Figure 7 corresponds to the rotation of the drum to the ejection position;
- Figure 8 corresponds to the liquid ejection phase;
- Figure 9 shows a position of the drum during the return to its rest position;

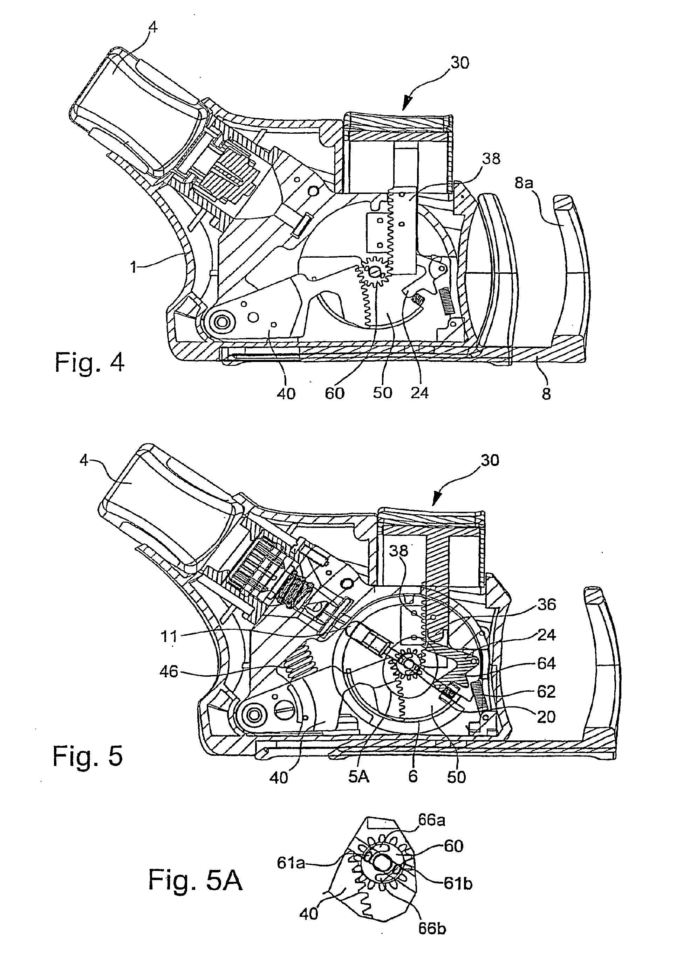

- Figures 5A to 9A show the different positions of the drive members in the phases corresponding to Figures 5 to 9;

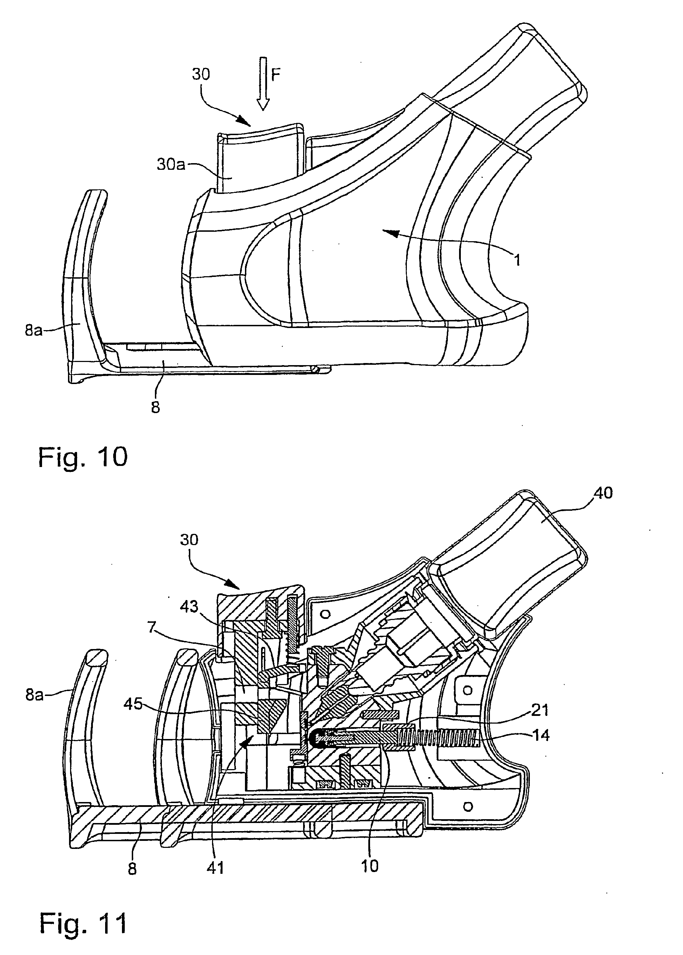

- Figure 10 shows a side view of a second embodiment of the invention;
- Figure 11 is a cross-section in the plane of symmetry of the apparatus shown in Figure 10 in the rest position;

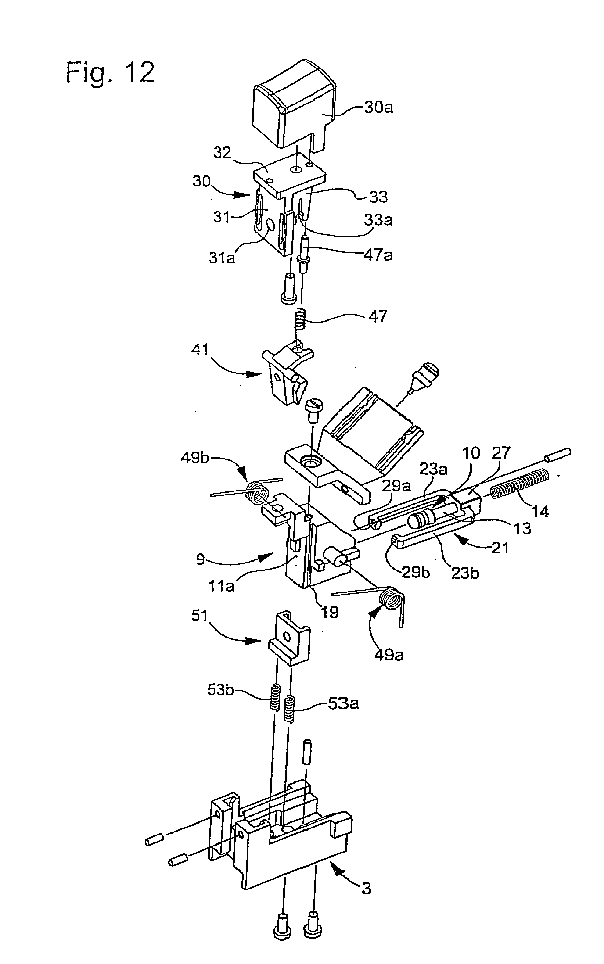

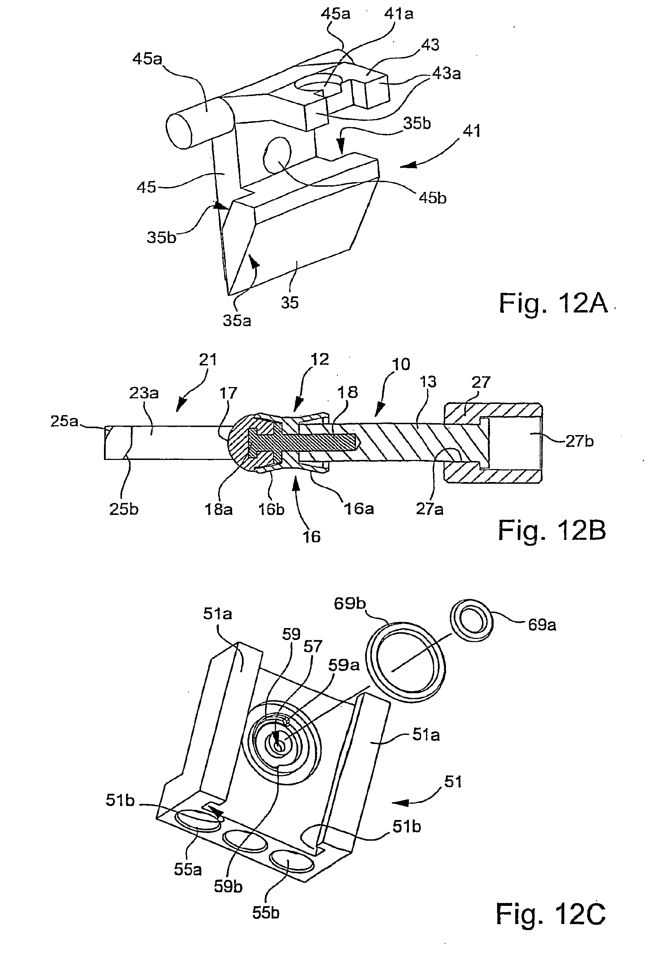

- Figure 12 is an exploded perspective view of the apparatus shown in Figure 10;
- Figures 12A and 12C are enlarged diagrams of two elements of the mechanism from another angle;
- Figure 12B is a cross-section of another element of the mechanism;

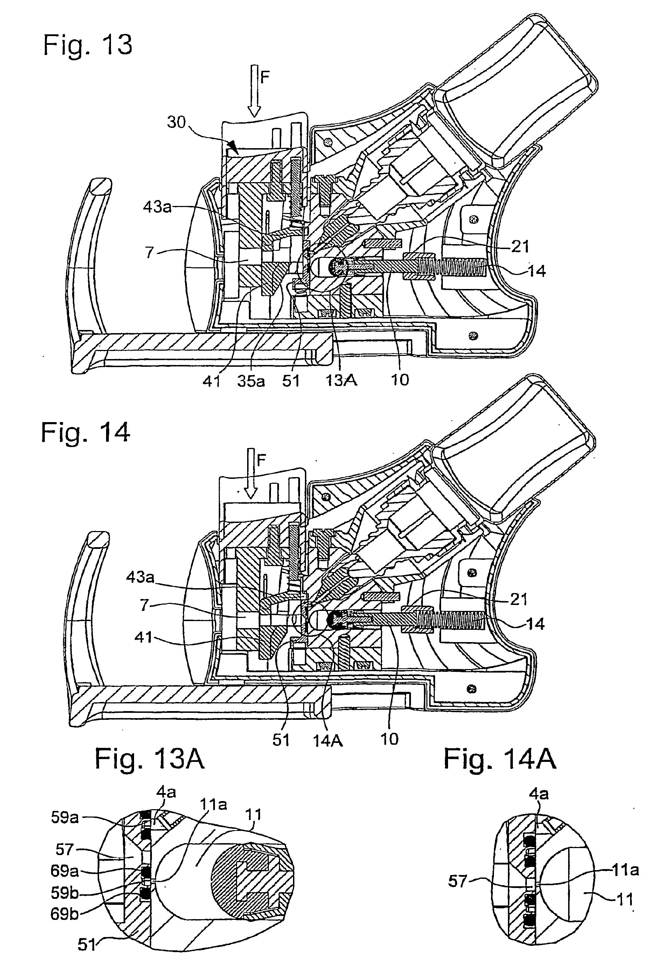

- Figure 13 corresponds to the suction phase of a determined quantity of liquid;
- Figure 13A is an enlarged diagram of the valve during the liquid suction phase;
- Figure 14 shows the phase during which the valve passes into the liquid ejection phase;
- Figure 14A is an enlarged diagram of the valve during the liquid ejection phase;

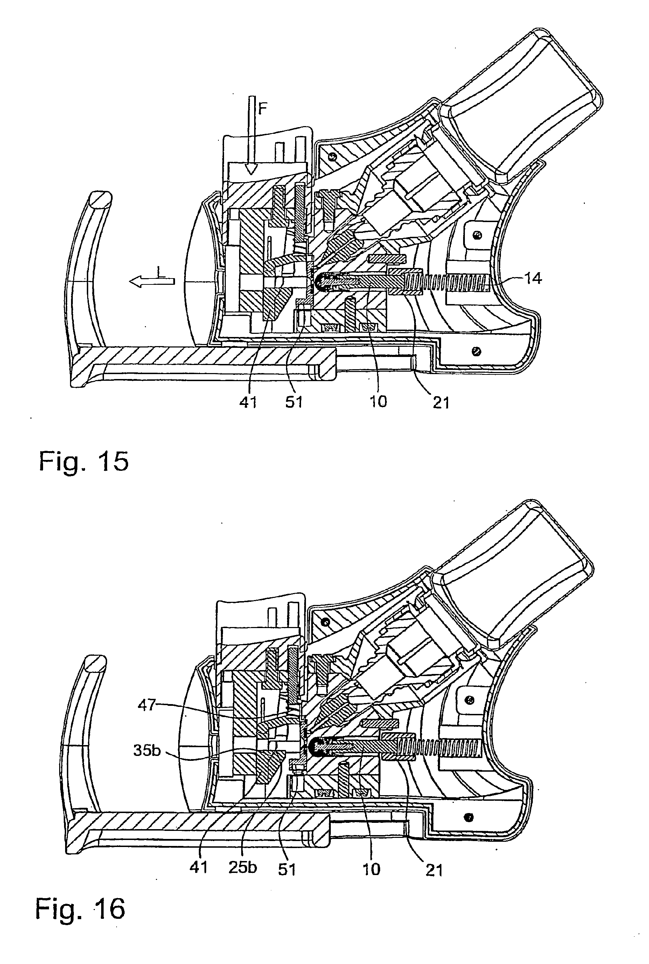

- Figure 15 shows the liquid ejection phase;
- Figures 16, 17, 18 show the return of the apparatus to the rest position; and

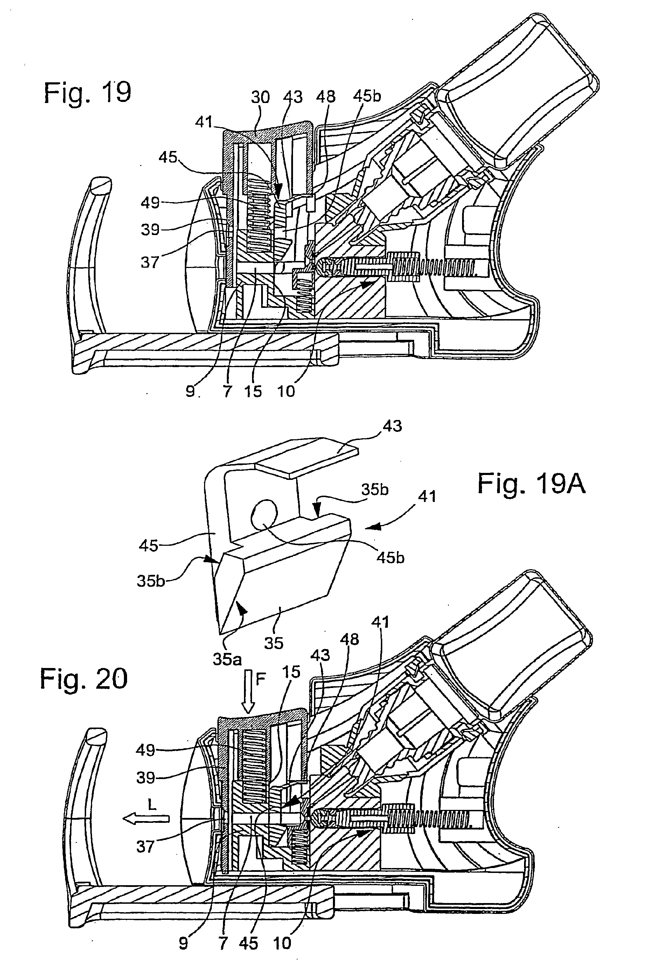

- Figures 19, 19A and 20 respectively show in the rest position and at the end of ejection a variant illustrated with the second embodiment.

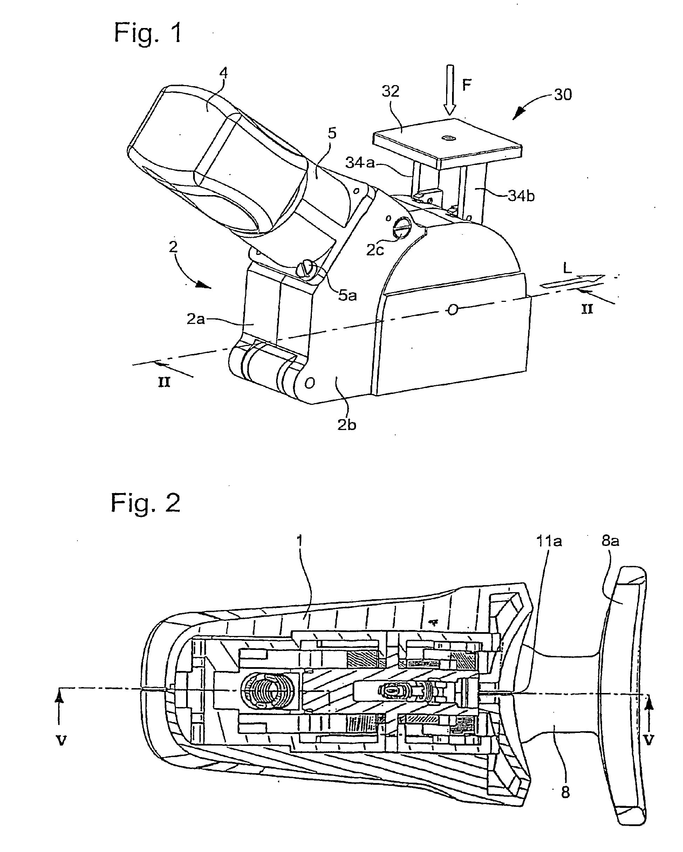

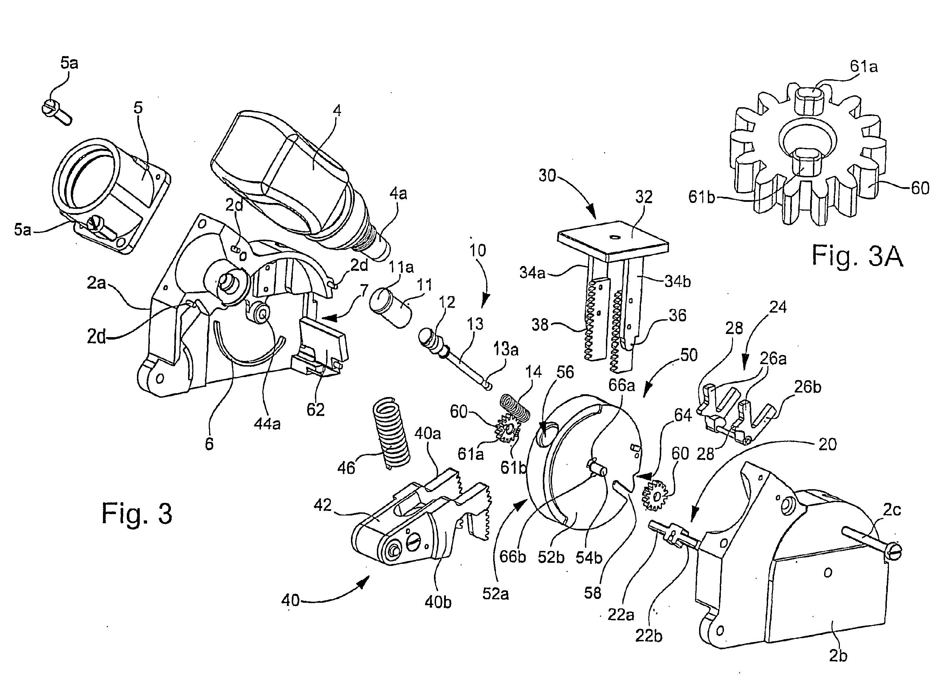

In Figure 1, Figures 1 and 10 show, in perspective, two embodiment examples of a dispensing apparatus according to the invention. In Figure 1, the apparatus includes an external cover 1 marking the mechanism of a second embodiment which will be described hereinafter where the external cover has been removed, one can see that externally the apparatus includes a housing 2 formed of two shells 2a, 2b assembled by a screw 2c after positioning the contact surfaces by means of pins 2d visible in the exploded view of Figure 3, to which reference will also be made in the description hereinafter. The liquid, which will have to be ejected from the apparatus in the direction of double arrow L, is contained in a receptacle 4 which, in this example, is a bottle ending in a feed nozzle 4a. Bottle 4 is secured to the apparatus by means of an adjustable clamp 5, to shells 2a, 2b by means of screws 5a. In Figure 1, it can also be seen that actuator 30, the actuation of which by a force F is effected in a substantially perpendicular direction to the direction of ejection of the liquid. In this embodiment, the actuator take the form of a push button and is generally U-shaped with a head 32 extended by two branches 34a, 34b, the construction and functions of which will be described hereinafter.

Reference will also now be made to Figure 4, in which external cover 1 has been kept, but shell 2b and drum 50 have been removed. Drum 50 forms, with the parts which drive it in one direction or another, the main mobile element of the mechanism according to the invention. Drum 50 includes on each of its flanks 52a, 52b studs 54a, 54b rotatably mounted in bearings 44a, 44b provided in the inner faces of shells 2a, 2b. Drum 50 also includes at its periphery an opening 56 corresponding to a through passage in which dosing chamber 11 will be mounted, provided with an ejection orifice 11a, a plunger 10 comprising a head 12, and a rod 13 having a groove 13a at its end. The particular structure of head 12, which contributes to the precision of the quantity of liquid ejected and to the non-contamination of the chamber by external polluting agents will be explained in more detail with reference to the second embodiment.

The drum also includes a slit 58 in which two lateral arms 22a, 22b of a staple 20 are engaged, said staple being snapped into groove 13a of rod 13, by compressing a spring 14 mounted on rod 13 of plunger 10, when said staple 20 is moved, from the bottom of slit 58 to the edge of drum 50. The movement of staple 20 is achieved by a double lever 24, articulated in its median part in shells 2a, 2b, each lever including an arm 26a pressing on each lateral arm 22a, 22b of staple 20. Each arm 26a of double lever 24 also includes a snug 28, allowing a safety catch 62 to be manoeuvred.

In proximity to slit 58, drum 50 also includes a notch 64 in which safety catch 62 will be engaged, the function of said catch being described hereinafter within the scope of the description of the working of the apparatus. Finally, drum 50 includes on each of its flanks 52a, 52b, two bean-shaped holes 66a, 66b, the function of which is explained hereinafter.

On each of studs 54a, 54b of drum 50 there is mounted a pinion 60, each pinion including along its axis two pins 61 a, 61 b, more clearly visible in enlarged Figure 3A. When a pinion 60 is mounted on a stud 54a, 54b of the drum, pins 61 a, 61 b are engaged in holes 66a, 66b, such that, when pinion 60 is driven in rotation, it has a small angle of shake during which drum 50 is not driven in rotation.

In Figure 4, it can be seen that pinions 60 mesh with the toothings, on the one hand, of actuator 30, and of a return member 40 on the other hand.

As indicated at the beginning, the actuator includes symmetrical branches 34a, 34b, the spacing of which substantially corresponds to the width of the drum. Each branch 34a, 34b is formed of an external part ending in a stop member 36, for manoeuvring arms 26b of lever 24, and of an internal part formed by a straight rack 38 extending on either side of stop member 36 in the longitudinal direction of branches 34a, 34b.

Return member 40 is formed by a double pivoting rack including two branches 40a, 40b connected by a bridge 42, the pivoting rack being articulated in shells 2a, 2b of housing 2. A return spring 46 allows the double rack to be kept in the low position when there is no pressure exerted on actuator 30 and to return it to this position when the actuator is released after having exerted pressure on the latter.

Finally, it can be seen that the inner surfaces of shells 2a, 2b each include a cam 6 having the shape of an arcuate rib. The end 22a, 22b of the lateral arms of staple 20 are capable of sliding on the external contour of rib 6 in order to keep spring 14 compressed during the rotation of drum 50 between the filling position and the ejection position. In the example illustrated cam 6 extends over an angle of approximately 120°.

The parts which have just been described, essentially with reference to the exploded view of Figure 3, appear at least partially in the cross-section of Figure 2 where the mechanism is shown with its external cover 1 and a sliding member 8 for adjusting the distance between the ejection orifice and an eyepiece 8a located at its end. Sliding member 8 and eyepiece 8a are shown in two end positions in Figure 4. Figure 3 also shows the cross-section line V-V corresponding to Figures 5 to 9 which will now enable the operation of the mechanism to be explained.

The operation of this first embodiment is now described with reference to Figures 5 to 9.

### Rest position (Figures 5 and 5A)

No pressure is exerted on actuator 30. Safety catch 62 is engaged in notch 64 of drum 50 and the orifice of dosing chamber 11 is opposite the nozzle of receptacle 4. Spring 46 rests on return rack 40, keeping pins 61a, 61 b in the low position in holes 66a, 66b. The two ends of lever 24 are abutting respectively against stop member 36 and staple 20. As the plunger head is pressed against the bottom of the dosing chamber, receptacle 4 is perfectly insulated from the external environment, and leaves no ullage.

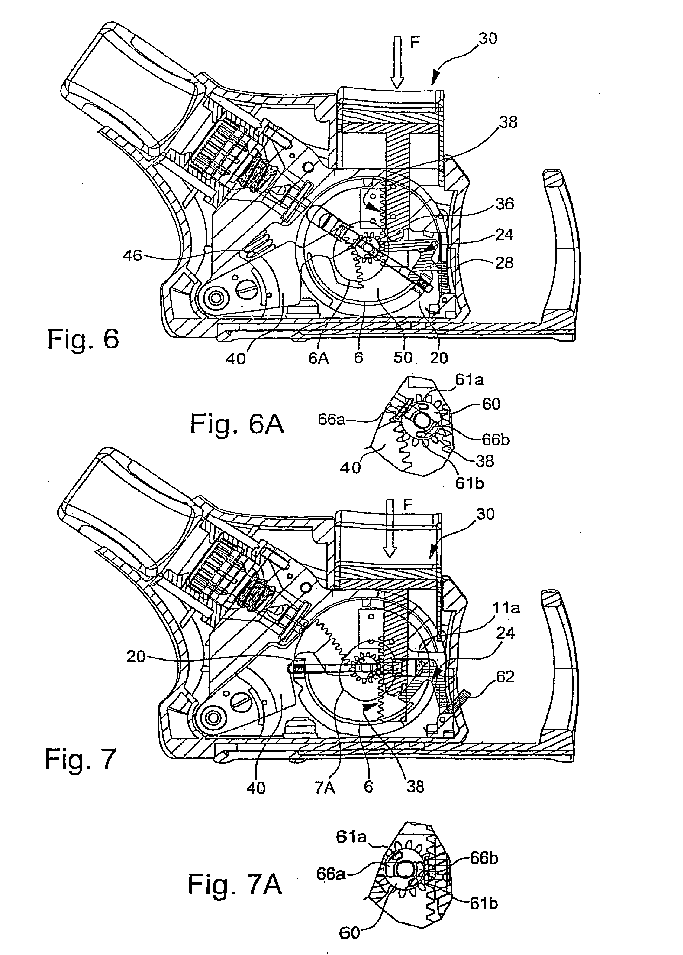

### Dosing chamber filling position (Figures 6 and 6A)

By exerting a pressure F on actuator 30, stop member 36 tips lever 24, and rack 38 drives pinion 60 to a high position in which pins 61 a, 61 b do not drive drum 50. In this step lever 24 pulls plunger 10 thus sucking up the liquid from bottle 4 to fill the dosing chamber to a position where staple 20 is placed behind cam 6. At this moment snug 28 of lever 24 pushes back safety catch 62 releasing drum 50. In this phase, spring 46 starts to be compressed.

### Passage into the ejection position (Figures 7 and 7A)

By continuing to exert pressure F on actuator 30, rack 38 drives pinion 60 which itself rotates drum 50, by means of pins 61 a, 61 b which rest on one end of holes 66a, 66b. During this rotation, staple 20 follows via its lateral arms the external contour of the rib forming cam 6. Figure 7 shows the position just preceding ejection, orifice 11 a of dosing chamber 11 being substantially on the axis of ejection. Rack 40 then exerts maximum compression on spring 46.

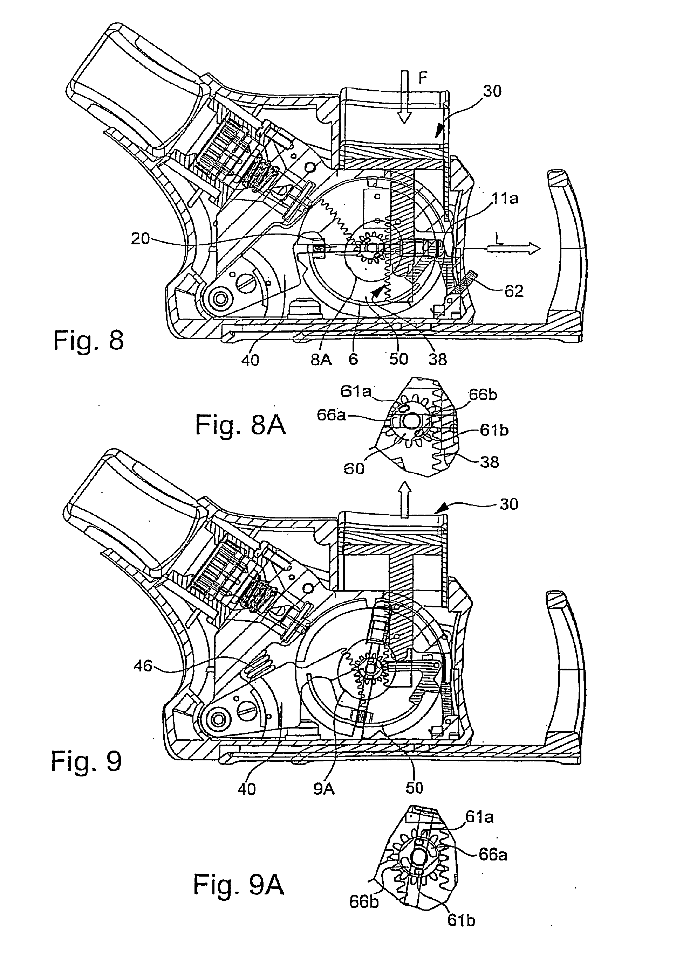

### The ejection position (Figures 8 and 8A)

By exerting an additional pressure, the lateral arms of staple 20 go beyond the end of cam 6 so that the staple is no longer held. Return spring 14 of plunger 10 then pushes the plunger head to the end of dosing chamber 11 to eject the liquid. In this phase it will be observed that the pressure with which the liquid is ejected depends solely upon the characteristics chosen for spring 14, and in no way upon those of return spring 46, nor the manner in which the user exerts force F.

It will also be observed that, if the user does not reach this ejection position by releasing pressure F during filling or rotation of the drum, the dosing chamber is returned to its initial position and the unused product is re-injected into the receptacle. This constitutes a certain advantage when the product is a medicinal one whose price is generally high.

### Return to the rest position (Figures 9 and 9A)

By releasing the pressure after ejecting the liquid, return spring 46 tips rack 40 in the opposite direction driving drum 50 via pinion 60 whose pins 61, 61 b are stopped at the other end of holes 66a, 66b. At the end of rotation, drum 50 again occupies the position shown in Figure 5. The apparatus is again in position for a new use.

With reference now to Figures 10 to 18, a second embodiment will be described hereinafter, in which the mobile element is formed by a valve 51, able to be moved by the action of the actuator, along the same direction as the latter, to place, in a first phase, the receptacle containing the liquid in communication with the dosing chamber, then, in the second phase, in communication with the exterior.

The side view of Figure 10 shows a dispensing apparatus with the same external appearance as the previously described apparatus, and wherein the entire mechanism is masked by external cover 1, leaving only actuator 30 visible, itself including an external cover 30a, bottle 4 forming the receptacle containing a liquid, for example an ophthalmic solution, and slide 8 with its eyepiece 8a.

The actual mechanism will now be described, referring essentially to Figures 11 and 12. It can be seen that the mechanism is assembled by means of frame 3 for receiving a unit 9 in which the dosing chamber is formed, more clearly visible in Figure 13A. Actuator 30 includes, perpendicular to its head 32, a plate 31 provided with an aperture 31a, and perpendicular to said plate a thick rib 33 including a snap-fitting groove 33a for a tipping element 41, having a reverse L shape, an enlarged perspective of which is shown in Figure 12A. L-shaped element 41 forms the control member which acts, in a first movement phase of actuator 30, on means for actuating plunger 10 against the action of a return spring 14, and in a second phase on a valve 51 able to move in the same direction as actuator 30, against the action of return springs 53a, 53b disposed between said valve 51 and frame 3. As can be seen more clearly in enlarged Figure 12A, L-shaped tipping element 41 includes a recess 41 a, for receiving one end of a helical spring 47 and the other end of which is held abutting against thick rib 33 by means of a spacer 47a.

Spring 47 is intended to hold element 41 abutting against a face of plate 31 during the active phase of actuator 30, then to be compressed during the return to the rest phase to allow said element 41 to tip and move aside behind the control member of plunger 10. The junction between the small branch 43 and large branch 45 includes on each of its edges pivots 45a allowing rib 33 to snap fit into groove 33a. Large branch 45 includes, in its substantially median part, an aperture 45b opposite aperture 31 a of plate 31. At its base, branch 45 includes a corner shape 35 defining on the exterior an inclined plane 35a and in the interior two inclined ramps 35b parallel to inclined plane 35a and the width of which is substantially the same as the length of pivots 45a.

Valve 51, which can move in sliding channels 19 of unit 9 is described in more detail with reference to enlarged Figures 12C and 13A. It is formed of a parallelepiped body including two edges 51 a in which two grooves 51 b are formed, allowing sliding on slide ways 19 of unit 9. Its base includes an edge which includes small circular recesses 55a, 55b directed downwards to position return springs 53a, 53b.

The surface delimited by the two edges 51 a and pressed against the surface opposite unit 9, includes at its centre an aperture 57 and a channel 59 whose ends 59a, 59b are located on either side of aperture 57 in the plane of symmetry of valve 51. Aperture 57 is surrounded by an inner O ring joint 69a and channel 59 by an outer O ring joint 69b, these joints 69a, 69b assuring sealing during movement of the valve. The longitudinal cross-section of Figure 13A shows the filling position in which nozzle 4a of receptacle 4 is placed in communication with orifice 11 a of dosing chamber 11, by ends 59a, 59b of channel 59, which preferably has the shape of the arc of a circle. Figure 14A shows the ejection position in which aperture 57 of the valve is brought opposite orifice 11 a of dosing chamber 11, nozzle 4a then be blocked by the surface of valve 51.

The actuating means for plunger 10, shown in cross-section in Figure 12B is formed by a clamp 21 including two large arms 23a, 23b ending in two lugs 29a, 29b the spacing of which substantially corresponds to the width of unit 9. The large arms 23a, 23b are connected by a base 27 including a hole 27a for securing rod 13 of plunger 10 and a recess 27b for positioning return spring 14. Lugs 29a, 29b each include two chamfers 25a, 25b having substantially the same inclination as inclined planes 35a, 35b of L-shaped tipping element 41. As will be explained hereinafter for the operation of the device, chamfers 25a, 25b each co-operate with inclined planes 35a, 35b, in a first phase, to act on plu nger 10 filling dosing chamber 11 and, in the second phase, to allow the device to return to the rest position.

The cross-section of Figure 12B also shows a new design of plunger head 12 providing both greater precision in the suction/ejection of a determined quantity of liquid, and safety as regards contaminating elements able to come from the exterior through the sliding cylinder of the plunger. Plunger head 12 is formed of two parts 16, 17 assembled by an assembling member 18 having the form of a rod provided with a head 18a and a collar. The first part 16 has the shape of an inverted double cone 16a, 16b through which assembling member 18 passes, to secure it in rod 13, on the side of cone 16a. This first part 16 is made of a hard plastic material, such as polypropylene (OP) or polyethylene (PE). The second part 17 is formed by a sealing gasket 17, made of a flexible plastic material, such as a thermoplastic elastomer (TPE) or silicon, disposed in the second inverted cone 16b to fit into head 18a of pin 18. The external part of gasket 17 has a hemispheric shape substantially corresponding to the shape of the bottom wall of the dosing chamber, as can be seen in Figures 13A and 14A. This design allows no ullage to be left during ejection of the liquid, and thus a precise quantity of liquid to be ejected, which is particularly important for medicinal products, and particularly ophthalmic solutions. The lips (not referenced) of inverted double cones 16a, 16b enable external polluting agents to be confined at the depression of their junction.

Plunger 10 which has just been described, for this second embodiment is also that found in the first embodiment described hereinbefore. It is clear that this plunger constitutes a preferred embodiment allowing the objectives of precision and sterility to be achieved for the dispensing apparatus according to the invention, but other types of plunger can be used without departing from the scope of the mechanisms which have just been described, and the operation of which is explained in more detail with reference to Figures 13 to 18.

### Filling position (Figures 13 and 13A)

From the rest position shown in Figure 11, exerting a pressure F on the head of actuator 30, the inclined plane 35a of L-shaped tipping element 41 slides the corresponding chamfer 25a of clamp 21, pushing back plunger 10 and compressing spring 14. In this position the base 4a of the receptacle is in communication with the orifice of dosing chamber 11 via channel 59 and enables dosing chamber 11 to be filled.

### Passage into the ejection position (Figures 14 and 14A)

By continuing to exert pressure F, the ends 43a of small arm 43 of the L-shaped tipping element press on valve 51, compressing return springs 53a, 53b to move said valve 51 to a position in which its aperture 57 is opposite orifice 11 a of dosing chamber 11. In this phase, the plunger spring remains compressed.

### Ejection position (Figure 15)

By continuing to press on the actuator, L-shaped element 41 releases clamp 21, and allows the liquid to be ejected via the action of return spring 14.

As indicated in the first embodiment, if the action on the actuator is interrupted, the quantity of liquid present in the chamber is re-injected into the receptacle.

### Return to the rest position (Figures 16, 17 and 18)

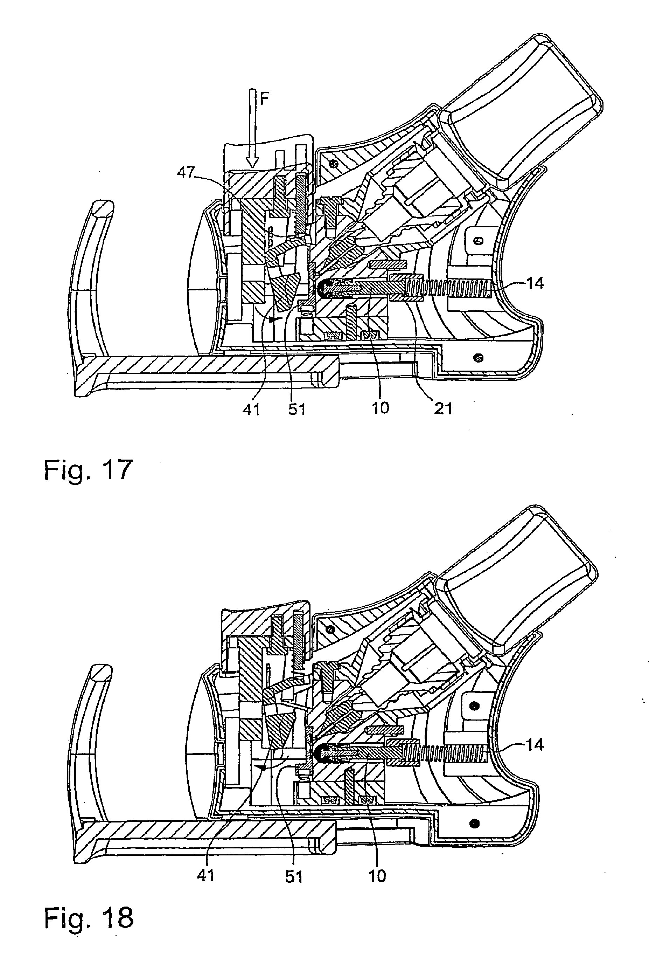

By releasing the pressure on the actuator, in a first phase (FIG.16) the second inclined plane 35b of L-shaped element 41 is positioned behind the corresponding inclined plane 25b of clamp 21. In a second phase (FIG. 17), L-shaped element 41 tips compressing spring 47, and in a second phase (FIG. 18), L-shaped element 41 is returned to its initial position by spring 47. This return to the rest position is actuated by springs 49 compressed via the action of the actuator.

Figures 19, 19A and 20 show a variant of a second embodiment wherein a modified element is also applicable to the first embodiment.

In Figure 19, which shows the apparatus in the rest position, it can be seen that actuator 30 is extended in the direction in which pressure F is exerted by a panel 39 insulating through passage 7 from the external environment when the apparatus is not being used. Panel 39 is provided with an aperture 37 which is placed opposite through passage 7 when the ejection position is reached, as shown in Figure 20. This variant allows conditions of sterility to be increased, even if in the first embodiment the flank of the drum already forms, in the rest position, a first means for insulating the whole of the apparatus from the external environment.

Figure 19 also shows variants relative to the second embodiment whose object is to make the apparatus according to the invention more economical.

The two actuator return springs 49a, 49b are replaced by a single spring 49 disposed between the inner face of actuator 30 and unit 9 of frame 3.

It can also be seen that the body of actuator 30, its external cover 30a and panel 39 are made in a single piece. The same is true of plunger 10 as regards clamp 21 and rod 13.

Again with reference to Figure 19A, it can also be seen that L-shaped tipping element 41 has been modified and simplified, while fulfilling the same function, with, however, slightly different kinematics. Small branch 43 has been thinned so as to have sufficient flexibility to allow the L-shaped element to more aside upon return to the rest position; spring 47 has thus been omitted. It can also be seen that L-shaped tipping element 41 no longer includes pivots 45a, 45b. Said tipping element 41 is driven in translation by actuator 30 by having the end of its small arm 43 gripped in an extension 48 of the actuator, whereas the large arm 45, which still has a corner shaped end 35 with the two inclined planes 35a, 35b, slides over a vertical wall 15 of unit 9 when a pressure F is exerted on actuator 30.

It is clear that the devices described are arranged for multi-dose applicaitons, i.e. applications in which doses are repeatedly drawn from a supply and repeatedly ejected. It is also clear that the devices are exemplified with features suitable for eye treatment applications. Typical parameters for this application will be given below although the invention shall not be regarded as limited to this application or any such exemplified parameter. A typical single dose volume for delivery to the eye can be less than 100 microliter, preferably less than 50 microliter, preferably less than 25 microliter, preferably less than 15 and most preferably less than 10 microliter. Generally the volume is at least 1, preferably at least 2 and most preferably at least 3 microliter. The liquid receptacle or supply line preferably has the capacity to deliver a plurality of such doses. A suitable speed for the stream of drops or jet ejected should be a balance between on one hand enough linear momentum to traverse an air gap between opening and target, without gravity assistance, and to travel fast enough not be obstructed by blinking and on the other hand not so fast as to cause inconvenient sensible impact on the eye. The ideal speed is to some extent dependent on the drop size used but as a general rule the drops should be able to traverse at least 1 cm, preferably at least 3 and most preferably at least 5 cm through air by own momentum, incorporating reasonable distances between opening and target. A suitable lower speed limit when leaving the opening is 1, m/s, preferably at least 5 m/s and most preferably at least 10 m/s. Generally the speed is lower than 200 m/s and preferably lower than 100 m/s. A suitable drop size so defined should be sufficient not to be retarded too quickly and not to be easily redirected, e.g. to be inhaled, and preferably has a minimum diameter of 20 micron, preferably not less than 50 micron and most preferably at least 100 microns. Normally the size is less than 2000 micron and preferably less than 1500 micron. The stream may take the form of a shower or spray of atomized liquid droplets but preferably the stream is narrow and fairly coherent although even such a stream tend to break up into individual droplets after a certain time of distance. The above given values are intended to relate to spherical droplets and for multiple droplets to the weight average of particle diameters. A coherent stream tends to break up into droplets of a diameter of roughly double the diameter of the stream. Accordingly suitable opening diameters for the containers are about half the above given drop diameters or roughly between 10 and 1000 microns, preferably between 20 and 800 microns. The above considerations are fairly independent of liquid viscosity and tend to apply both for solutions and ointments. It is desirable that the whole dose can be delivered in a time shorter than the blink reflex time, i.e. in a time shorter than about 150 ms, preferably shorter than 100 ms and most preferably shorter than 75 ms.

## Claims

1. A dispensing apparatus for a liquid product, the apparatus comprising a) a housing (2) or frame (3), b) a receptacle (4) for the liquid with a feed nozzle (4a) arranged substantially stationary with respect to the housing or frame, c) a dosing chamber (11) having an orifice (11a), d) a mechanism (50) arranged to allow at least ejection of liquid through the orifice and e) a through passage (7) arranged to allow the ejected liquid to pass In a direction different from the feed nozzle or opening **cha racterized in**, i) that the mechanism (50) comprises a mobile element (50,51) arranged movable with respect to the housing or frame between at least a first position in which the orifice of the dosing chamber and the feed nozzle or opening are in flow communication and a second position in which the orifice and the through passage are in flow communication, and ii) that the mechanism (50) is arranged to allow aspiration of liquid through the orifice when the mobile element is in the first position and ejection of liquid through the orifice when the mobile element is in the second position.

2. The apparatus of claim 1, **characterized in that** the mobile element (50,51) is arranged to move or carry the dosing chamber (11) between a filling position, when the mobile element is in the first position, and an ejection position, when the mobile element is in the second position.

3. The apparatus of claim 1, **characterized in that** the dosing chamber comprises a substantially cylindrical barrel, defining a concentric barrel axis.

4. The apparatus of claim 3, **characterized in that** the mobile element is arranged to move or carry the dosing chamber in a rotational movement around a rotation axis (54b) different from the barrel axis.

5. The apparatus of claim 4, **characterized in that** the rotation axis is (54b) substantially perpendicular to the barrel axis.

6. The apparatus of claim 1, **characterized in that** the mobile element comprises a valve (51), having a passage (59) and a hole (57) or the through passage (7), the passage being arranged to connect the nozzle or opening with the orifice when the mobile element is in the first position and to align the orifice with the hole or the through passage when the mobile element Is In the second position.

7. The apparatus of claim 4, **characterized in that** the passage is arranged to be shut off when the mobile element is In the second position.

8. The apparatus of claim 7, **characterized in that** the passage is shut off in both ends.

9. The apparatus of claim 6, **characterized in that** the dosing chamber is arranged substantially fixed with respect to the housing or frame.

10. The apparatus of claim 1, **characterized in that** the mechanism is arranged to perform in sequence the aspiration of liquid In the first position, the movement of the mobile element to the second position, the ejection of liquid in the second position.

11. The apparatus of claim 10, **characterized in that** the mechanism is arranged to perform the aspiration by retraction of a pump member against a return spring and to perform the ejection by release of the return spring.

12. The apparatus of claim 10, **characterized in that** the mechanism is arranged to allow return the mechanism to the start or rest positon after liquid election.

13. The apparatus of claim 12, **characterized in that** the mechanism is arranged to allow return of the mechanism to the start or rest position also before liquid ejection.

14. The apparatus of claim 13, **characterized in that** the mechanism is arranged to reinject the aspirated liquid in the receptacle (4) If the return takes place before ejection.

15. The apparatus of claim 12, **characterized in that** a return member (40) is arranged to blas the mechanism towards the start or rest position.

16. The apparatus of claim 1 or 10 to 15, **characterized in that** a at least one actuator (30) is arranged to operate the mechanism (50,51).

17. The apparatus of claim 16, **characterized in that** the actuator is arranged to be manoeuvred by application of manual force (F).

18. The apparatus of claim 16, **characterized in that** the actuator is arranged perform a substantially continuous movement during which the mechanism performs at least the aspiration step and the movement of the mobile element between the first position and the second position.

19. The apparatus of claim 18, **characterized in that** the actuator is arranged to include in the substantially continuous movement also the ejection step for the mechanism.

20. The apparatus of claim 19, **characterized in that** the actuator is arranged to give a tactile feed-back immediately before the ejection step in the continuous movement.

21. The apparatus of claim 1, **characterized in that** the dosing chamber comprises a substantially cylindrical barrel, defining a concentric barrel axis and having a substantially constant cross-section area perpendicular to the barrel axis, and a plunger inserted in the barrel and being movable along the barrel axis.

22. The apparatus of claim 21, **characterized in that** the orifice has a substantially smaller cross-section area than the barrel.

23. The apparatus of claim 27, **characterized in that** the length of the orifice in the liquid flow direction is substantially shorter than the plunger movement during aspiration and/or ejection.

24. The apparatus of claim 23, **characterized in that** the orifice length to plunger movement length is less than 1:5, preferably less than 1:10 and most preferably less than 1:20.

25. The apparatus of claim 1, **characterized in that** the orifice is arranged to create a liquid spray.

26. The apparatus of claim 1, **characterized in that** the orifice is arranged to create a substantially coherent stream.

27. The apparatus of claim 1, **characterized in that** in the second position the orifice Is arranged to eject liquid substantially directly into the air.

28. The apparatus of claim 27, **characterized in that** the through passage is substantially wider than the width of the orifice.

29. The apparatus of claim 28, **characterized in that** any apparatus part in front of the orifice (11a) is substantially wider than the orifice (11a).

30. The apparatus of claim 1, **characterized in that** it comprises an eye piece or eye cup designed to define a predetermined distance to the orifice.

## Patentansprüche

1. Spendervorrichtung für ein flüssiges Produkt, wobei die Vorrichtung a) ein Gehäuse (2) oder einen Rahmen (3), b) einen Aufnahmebehälter (4) für die Flüssigkeit mit einer Zuführungsdüse (4a) im Wesentlichen stationär angeordnet bezüglich des Gehäuses oder Rahmens, c) eine Dosierkammer (11) mit einer Öffnung (11a), d) einen Mechanismus (50) angeordnet, um mindestens einen Ausstoß von Flüssigkeit durch die Öffnung zu ermöglichen, und e) einen Durchlass (7), angeordnet, um zu ermöglichen, dass die ausgestoßene Flüssigkeit sich in eine Richtung bewegt, die von der Zuführungsdüse oder dem Durchgang verschieden ist, umfasst, **dadurch gekennzeichnet,** i) dass der Mechanismus (50) ein bewegliches Element (50, 51), ortsveränderlich angeordnet bezüglich des Gehäuses oder des Rahmens zwischen mindestens einer ersten Position, in welcher sich die Öffnung der Dosierkammer und die Zuführungsdüse oder der Durchgang in Fließverbindung befinden, und einer zweiten Position, in welcher sich die Öffnung und der Durchlass in Fließverbindung befinden, umfasst und ii) dass der Mechanismus (50) angeordnet ist, um das Aufsaugen von Flüssigkeit durch die Öffnung, wenn sich das bewegliche Element in der ersten Position befindet, und den Ausstoß der Flüssigkeit durch die Öffnung, wenn sich das bewegliche Element in der zweiten Position befindet, zu ermöglichen.

2. Vorrichtung nach Anspruch 1, **dadurch gekennzeichnet, dass** das bewegliche Element (50, 51) angeordnet ist, um die Dosierkammer (11) zwischen einer Füllposition, wenn sich das bewegliche Element in der ersten Position befindet, und einer Ausstoßposition, wenn sich das bewegliche Element in der zweiten Position befindet, zu bewegen oder mitzuführen.

3. Vorrichtung nach Anspruch 2, **dadurch gekennzeichnet, dass** die Dosierkammer eine(n) im Wesentlichen zylindrische(n) Trommel bzw. Zylinder umfasst, die/der eine konzentrische Zylinderachse definiert.

4. Vorrichtung nach Anspruch 3, **dadurch gekennzeichnet, dass** das bewegliche Element angeordnet ist, um die Dosierkammer in einer Rotationsbewegung um eine Rotationsachse (54b), die von der Zylinderachse verschieden ist, zu bewegen oder mitzuführen.

5. Vorrichtung nach Anspruch 4, **dadurch gekennzeichnet, dass** die Rotationsachse (54b) im Wesentlichen senkrecht zu der Zylinderachse ist.

6. Vorrichtung nach Anspruch 1, **dadurch gekennzeichnet, dass** das bewegliche Element ein Ventil (51) umfasst, welches einen Kanal (59) und ein Loch (57) oder den Durchlass aufweist, wobei der Kanal angeordnet ist, um die Düse oder den Zugang mit der Öffnung zu verbinden, wenn sich das bewegliche Element in der ersten Position befindet, und die Öffnung mit dem Loch oder dem Durchlass in eine Linie zu bringen, wenn sich das bewegliche Element in der zweiten Position befindet.

7. Vorrichtung nach Anspruch 6, **dadurch gekennzeichnet, dass** der Kanal angeordnet ist, um abgeschlossen zu sein, wenn sich das bewegliche Element in der zweiten Position befindet.

8. Vorrichtung nach Anspruch 7, **dadurch gekennzeichnet, dass** der Kanal an beiden Enden abgeschlossen ist.

9. Vorrichtung nach Anspruch 6, **dadurch gekennzeichnet, dass** die Dosierkammer im Wesentlichen fixiert bezüglich des Gehäuses oder des Rahmens angeordnet ist.

10. Vorrichtung nach Anspruch 1, **dadurch gekennzeichnet, dass** der Mechanismus angeordnet ist, um nacheinander das Aufsaugen der Flüssigkeit in der ersten Position, die Bewegung des beweglichen Elements zu der zweiten Position, den Ausstoß von Flüssigkeit in der zweiten Position durchzuführen.

11. Vorrichtung nach Anspruch 10, **dadurch gekennzeichnet, dass** der Mechanismus angeordnet ist, um das Aufsaugen durch Zurückziehen eines Pumpenelements gegen eine Rückstellfeder durchzuführen und um den Ausstoß durch Freigabe der Rückstellfeder durchzuführen.

12. Vorrichtung nach Anspruch 10, **dadurch gekennzeichnet, dass** der Mechanismus angeordnet ist, um die Rückkehr des Mechanismus in die Start- oder Halteposition nach dem Flüssigkeitsausstoß zu ermöglichen.

13. Vorrichtung nach Anspruch 12, **dadurch gekennzeichnet, dass** der Mechanismus angeordnet ist, um die Rückkehr des Mechanismus in die Start- oder Halteposition auch vor dem Flüssigkeitsausstoß zu ermöglichen.

14. Vorrichtung nach Anspruch 13, **dadurch gekennzeichnet, dass** der Mechanismus angeordnet ist, um die aufgesaugte Flüssigkeit in den Aufnahmebehälter (4) zurückzuinjizieren, wenn die Rückkehr vor dem Ausstoß stattfindet.

15. Vorrichtung nach Anspruch 12, **dadurch gekennzeichnet, dass** ein Rückstellelement (40) angeordnet ist, um den Mechanismus in Richtung der Start- oder Halteposition zu neigen bzw. zu bringen.

16. Vorrichtung nach Anspruch 1 oder 10 oder 15, **dadurch gekennzeichnet, dass** wenigstens ein Betätigungselement (30) angeordnet ist, um den Mechanismus (50, 51) zu bedienen.

17. Vorrichtung nach Anspruch 16, **dadurch gekennzeichnet, dass** das Betätigungselement angeordnet ist, um durch Anwendung einer manuellen Kraft (F) bewegt zu werden.

18. Vorrichtung nach Anspruch 16, **dadurch gekennzeichnet, dass** das Betätigungselement angeordnet ist, um eine im Wesentlichen kontinuierliche Bewegung durchzuführen, während derer der Mechanismus mindestens den Ansaugeschritt und die Bewegung des beweglichen Elements zwischen der ersten Position und der zweiten Position durchführt.

19. Vorrichtung nach Anspruch 18, **dadurch gekennzeichnet, dass** das Betätigungselement angeordnet ist, um in die im Wesentlichen kontinuierliche Bewegung auch den Ausstoßschritt für den Mechanismus einzuschließen.

20. Verfahren nach Anspruch 19, **dadurch gekennzeichnet, dass** das Betätigungselement angeordnet ist, um eine fühlbare Rückmeldung unmittelbar vor dem Ausstoßschritt in der kontinuierlichen Bewegung zu geben.

21. Vorrichtung nach Anspruch 1, **dadurch gekennzeichnet, dass** die Dosierkammer eine(n) im Wesentlichen zylindrische(n) Trommel bzw. Zylinder umfasst, die/der eine konzentrische Zylinderachse definiert und eine im Wesentlichen konstante Querschnittsfläche senkrecht zu der Zylinderachse und einen Stempel, eingeführt in den Zylinder und beweglich entlang der Zylinderachse, aufweist.

22. Vorrichtung nach Anspruch 21, **dadurch gekennzeichnet, dass** die Öffnung eine wesentlich kleinere Querschnittsfläche als der Zylinder aufweist.

23. Vorrichtung nach Anspruch 22, **dadurch gekennzeichnet, dass** die Länge der Öffnung in Flüssigkeitsfließrichtung wesentlich kürzer ist als die Stempelbewegung während des Ansaugens und/oder des Ausstoßes.

24. Vorrichtung nach Anspruch 23, **dadurch gekennzeichnet, dass** die Öffnungslänge zu der Stempelbewegungslänge weniger als 1:5, vorzugsweise weniger als 1:10 und am stärksten bevorzugt weniger als 1:20, ist.

25. Vorrichtung nach Anspruch 1, **dadurch gekennzeichnet, dass** die Öffnung angeordnet ist, um ein Flüssigkeits-Spray zu erzeugen.

26. Vorrichtung nach Anspruch 1, **dadurch gekennzeichnet, dass** die Öffnung angeordnet ist, um einen im Wesentlichen zusammenhängenden Strom zu erzeugen.

27. Vorrichtung nach Anspruch 1, **dadurch gekennzeichnet, dass** in der zweiten Position die Öffnung angeordnet ist, um Flüssigkeit im Wesentlichen direkt in die Luft auszustoßen.

28. Vorrichtung nach Anspruch 27, **dadurch gekennzeichnet, dass** der Durchlass wesentlich weiter ist als die Breite der Öffnung.

29. Vorrichtung nach Anspruch 28, **dadurch gekennzeichnet, dass** jeglicher Vorrichtungsteil vor der Öffnung (11a) wesentlich weiter ist als die Öffnung (11a).

30. Vorrichtung nach Anspruch 1, **dadurch gekennzeichnet, dass** sie ein Augenstück oder einen Augenbecher umfasst, das/der konstruiert wurde, um einen vorbestimmten Abstand zu der Öffnung zu definieren.

## Revendications

1. Appareil de distribution pour produit liquide, l'appareil comprenant a) un boîtier (2) ou châssis (3), b) un réceptacle (4) pour le liquide, une buse d'alimentation (4a) étant agencée sensiblement fixe par rapport au boîtier ou châssis, c) une chambre de dosage (11) ayant un orifice (11a), d) un mécanisme (50) agencé pour permettre au moins l'éjection de liquide via l'orifice et e) un passage traversant (7) agencé pour permettre au liquide éjecté de passer dans une direction différente depuis la buse ou ouverture d'alimentation, **caractérisé en ce que** i) le mécanisme (50) comprend un élément mobile (50,51) agencé mobile par rapport au boîtier ou châssis entre au moins une première position dans laquelle l'orifice de la chambre de dosage et la buse ou ouverture d'alimentation sont en communication de fluide et une seconde position dans laquelle l'orifice et le passage traversant sont en communication de fluide, et **en ce que** ii) le mécanisme (50) est agencé pour permettre l'aspiration de liquide via l'orifice lorsque l'élément mobile est dans la première position et l'éjection de liquide via l'orifice lorsque l'élément mobile est dans la seconde position.

2. Appareil selon la revendication 1, **caractérisé en ce que** l'élément mobile (50,51) est agencé pour déplacer ou transporter la chambre de dosage (11) entre une position de remplissage, lorsque l'élément mobile est dans la première position, et une position d'éjection, lorsque l'élément mobile est dans la seconde position.

3. Appareil selon la revendication 2, **caractérisé en ce que** la chambre de dosage comprend un fût sensiblement cylindrique, définissant un axe concentrique de fût.

4. Appareil selon la revendication 3, **caractérisé en ce que** l'élément mobile est agencé pour déplacer ou transporter la chambre de dosage selon un mouvement de rotation autour d'un axe de rotation (54b) qui diffère de l'axe du fût.

5. Appareil selon la revendication 4, **caractérisé en ce que** l'axe de rotation (54b) est sensiblement perpendiculaire à l'axe du fût.

6. Appareil selon la revendication 1, **caractérisé en ce que** l'élément mobile comprend une soupape (51), ayant un passage (59) et un trou (57) ou le passage traversant (7), le passage étant agencé pour connecter la buse ou ouverture avec l'orifice lorsque l'élément mobile est dans la première position et pour aligner l'orifice avec le trou ou le passage traversant lorsque l'élément mobile est dans la seconde position.

7. Appareil selon la revendication 6, **caractérisé en ce que** le passage est agencé pour être fermé lorsque l'élément mobile est dans la seconde position.

8. Appareil selon la revendication 7, **caractérisé en ce que** le passage est fermé à ses deux extrémités.

9. Appareil selon la revendication 6, **caractérisé en ce que** la chambre de dosage est agencée sensiblement fixe par rapport au boîtier ou châssis.

10. Appareil selon la revendication 1, **caractérisé en ce que** le mécanisme est agencé pour mettre en oeuvre successivement l'aspiration de liquide dans la première position, le mouvement de l'élément mobile vers la seconde position, et l'éjection de liquide dans la seconde position.

11. Appareil selon la revendication 10, **caractérisé en ce que** le mécanisme est agencé pour mettre en oeuvre l'aspiration par retrait d'un élément de pompe à l'encontre d'un ressort de rappel et pour mettre en oeuvre l'éjection par libération du ressort de rappel.

12. Appareil selon la revendication 10, **caractérisé en ce que** le mécanisme est agencé pour permettre le retour du mécanisme vers la position de départ ou de repos après l'éjection du liquide.

13. Appareil selon la revendication 12, **caractérisé en ce que** le mécanisme est agencé pour permettre le retour du mécanisme vers la position de départ ou de repos également avant l'éjection de liquide.

14. Appareil selon la revendication 13, **caractérisé en ce que** le mécanisme est agencé pour réinjecter le liquide aspiré dans le réceptacle (4) si le retour se fait avant l'éjection.

15. Appareil selon la revendication 12, **caractérisé en ce qu'**un élément de rappel (40) est agencé pour solliciter le mécanisme vers la position de départ ou de repos.

16. Appareil selon la revendication 1 ou 10 ou 15,
**caractérisé en ce qu'**au moins un actionneur (30) est agencé pour faire fonctionner le mécanisme (50,51).

17. Appareil selon la revendication 16, **caractérisé en ce que** l'actionneur est agencé pour être manoeuvré par application d'une force manuelle (F).

18. Appareil selon la revendication 16, **caractérisé en ce que** l'actionneur est agencé pour mettre en oeuvre un mouvement sensiblement continu au cours duquel le mécanisme met en oeuvre au moins l'étape d'aspiration et le mouvement de l'élément mobile entre la première position et la seconde position.

19. Appareil selon la revendication 18, **caractérisé en ce que** l'actionneur est agencé pour inclure, dans le mouvement sensiblement continu, également l'étape d'éjection du mécanisme.

20. Appareil selon la revendication 19, **caractérisé en ce que** l'actionneur est agencé pour donner un retour tactile immédiatement avant l'étape d'éjection dans le mouvement continu.

21. Appareil selon la revendication 1, **caractérisé en ce que** la chambre de dosage comprend un fût sensiblement cylindrique, définissant un axe concentrique de fût et ayant une superficie, en section transversale perpendiculaire à l'axe du fût, sensiblement constante, et un piston inséré dans le fût et mobile selon l'axe du fût.

22. Appareil selon la revendication 21, **caractérisé en ce que** l'orifice a une superficie en section transversale sensiblement inférieure à celle du fût.

23. Appareil selon la revendication 22, **caractérisé en ce que** la longueur de l'orifice dans la direction d'écoulement du liquide est sensiblement plus courte que la course du piston au cours de l'aspiration et/ou de l'éjection.

24. Appareil selon la revendication 23, **caractérisé en ce que** le rapport entre la longueur de l'orifice et la longueur de la course du piston est inférieur à 1:5, de préférence inférieur à 1:10, et mieux inférieur à 1:20.

25. Appareil selon la revendication 1, **caractérisé en ce que** l'orifice est agencé pour créer un nébulisat de liquide.

26. Appareil selon la revendication 1, **caractérisé en ce que** l'orifice est agencé pour créer un courant sensiblement cohérent.

27. Appareil selon la revendication 1, **caractérisé en ce que**, dans la seconde position, l'orifice est agencé pour éjecter le liquide sensiblement directement dans l'air.

28. Appareil selon la revendication 27, **caractérisé en ce que** le passage traversant est sensiblement plus large que la largeur de l'orifice.

29. Appareil selon la revendication 28, **caractérisé en ce que** toute partie de l'appareil devant l'orifice (11a) est sensiblement plus large que l'orifice.

30. Appareil selon la revendication 1, **caractérisé en ce qu'**il comprend un oculaire ou une oeillère conçu(e) pour définir une distance prédéterminée jusqu'à l'orifice.
